# EUROPEAN PATENT SPECIFICATION

(11) **EP 3 159 049 B1**
(45) Date of publication and mention of the grant of the patent: **12.02.2020**
(21) Application number: 16195132.2
(22) Date of filing: 21.10.2016
(51) Int. Cl.: A63B 69/18, A63G 31/16, A63B 22/14, A63B 22/02, G06F 3/01

(54) **CURVE CORNERING SIMULATION DEVICE FOR PROVIDING THE FEELING OF SLIDING WHEN TURNING**
KURVENFAHRTSIMULATIONSVORRICHTUNG ZUR BEREITSTELLUNG DES GEFÜHLS VON GLEITEN IN KURVEN
DISPOSITIF DE SIMULATION DE VIRAGE DE COURBE POUR FOURNIR LA SENSATION DE GLISSER LORS DU VIRAGE

(30) Priority: 23.10.2015 KR 20150148371
(43) Date of publication of application: 26.04.2017
(73) Proprietor: Kang, Too Hwan, Seoul 04380 (KR)
(72) Inventor: Kang, Too Hwan, Seoul 04380 (KR)
(74) Representative: Szabo, Zsolt

(56) References cited:
- WO-A2-2012/111957
- ES-A1- 2 545 306
- KR-A- 20120 126 798
- US-A1- 2005 009 668
- US-A1- 2006 123 896

## Description

### TECHNICAL FIELD

The present invention relates to a curve cornering simulation device, and in particular to a curve cornering simulation device which allows for a straight traveling, a turning and cornering traveling in a virtual reality experience and is able to provide a friction feeling and a sliding feeling when turning.

### BACKGROUND ART

The present invention is referred to a curve cornering simulation device, and in particular to a curve cornering simulation device which allows for a straight traveling, a turning and cornering traveling in a virtual reality experience and is able to provide a friction feeling and a sliding feeling when turning. In recent years, various virtual reality experiencing devices which use applied virtual realities, are being disclosed. These devices are configured to convert any change to a virtual environment made using a program into a physical action through an action implementation device in such a way to connect a computer to the action implementation device, by which a user is able to recognize and feel a virtual situation like an actual situation.

As one of the representative virtual reality experiencing devices, a riding type virtual experiencing device is widely used, which is configured to provide a position displacement wherein a riding device made in the shapes of a chair, a vehicle, a helicopter or a horst changes its position in downward, upward, leftward and rightward directions based on the images shown on a large size screen in a state where a user is sitting. This riding type simulation device is equipped with a plurality of hydraulic or pneumatic cylinders at its lower side so as to vibrate or change, in the forward, backward, upward and downward directions, the position displacements of a chair or horse-shaped riding device. Various actions can implemented in such a way to provide a control signal to the aforementioned cylinders.

The riding type simulation device which uses a hydraulic or pneumatic cylinder, should convert a position control signal into a pneumatic or hydraulic control signal or should satisfy the compression condition of a pressure transfer medium, for which a facility becomes complicated, and a manufacture cost is high. Moreover, since a volume and a weight increase, an installation place may be limited. Since an operation reaction quickness is low, a reality may be degraded.

Moreover, if a hydraulic or pneumatic cylinder is used for a traveling type experiencing device, a reaction is slow, and a leftward or rightward rotation is not sensitive, and if such a device travels along a curved track, it is impossible to provide an actual sliding feeling which may occur during an actual rotation.

In the Korean patent laid-open number 10-2004-0088327 entitled "an actual feeling-like game device", the user's input of an input device, for example, a joystick, is reflected on the change in a game image, and a user's chair is provided with a rotational motion through gears, and a rotational motion can be made about a vertical shaft in such a way to use a motor and gears. In this device, it may take a long time until the input from a joystick is actually reflected on the user's chair. Since the chair simply provides a rotational motion, it does not feel like an actual traveling.

For example, if a driver hits to the maximum an accelerator when a vehicle turns at a corner in case of a rear wheel drive vehicle, the rear wheels may slip aside. In a state where a speed is not much reduced, the aforementioned technique may be used for an actual drive so as to turn at a corner. The aforementioned patent and currently available virtual reality experiencing devices are not equipped with any means for the sake of this sliding. In case of the hydraulic type, the device is configured to just move suddenly or intermittently.
Document KR20120126798A discloses a curve cornering simulation device comprising a foot board rotatable with respect to a rotary shaft, a driven track belt, a wheel configured for rotation on the top of the moving track belt, the steering bar controlling the rotation of the wheel, such that the foot board rotates accordingly.

### DISCLOSURE OF THE INVENTION

Accordingly, the present invention is made in an effort to resolve the aforementioned problems. It is an object of the present invention to provide an experiencing device which is able to provide an actual traveling-like feeling in a virtual reality experience.

To achieve the above objects, there is provided a curve cornering simulation device for providing the feelings of sliding and a friction when turning, which may include, but is not limited to, a foot board 3 which is rotatable with respect to a rotary shaft; a pair of pedals which are installed, with the rotary shaft being disposed between the pedals; and a caster 31 which is disposed under the pedals 1, wherein when a user steps on the pedals, a caster brake which is provided above the caster, comes down, thus interrupting the rotation of the caster, so the caster can contact with the upper surface of the belt of a track belt 7 wherein the belt moves outward of a pair of rollers, and if the foot pedals are stepped on, it can move in the direction where the foot pedals 1 are stepped on.

Moreover, the track belt is on the same straight line as the rotary shaft of the foot board 3 and is placed rotatable on a lower rotary shaft 11 which is not engaged, and a pair of lower casters 10 are installed at a lower end portion of the track belt 7, with the lower rotary shaft being disposed between the lower casters 10, by which it is pressed in the direction where a deviated weight occurs, thus being rotated.

In addition, the lower caster 10 is installed at the top of a base plate 12, and an intermediate frame 8 with a plurality of arms is placed on the lower rotary shaft 11, and a quadrangular frame 15 is engaged to the intermediate frame 8, and a track belt 7 is engaged to the quadrangular frame 15, and a lower caster 10 and a spring 9 support under the intermediate frame 8, and if a deviated weight is applied, the lower caster is pressed by the deviated weight.

Furthermore, a handle engaging part 2 is provided at an end portion of the foot board 3, and a handle shaft 20 stands at the handle engaging part 2, and a handle is provided at an end of the handle shaft 20, and the handle 17 is configured to move upward and downward, and both ends of the handle 17 are configured to move in different directions in cooperation with each other.

Moreover, the lower casters 10 and the upper casters 31 are configured to rotate in such a way that the casters are engaged to the inner sides of two support shafts, and a caster brake to which a spring is attached in the direction where the support shaft is engaged, is configured to move in the direction of the casters, and if the caster brake contacts with the casters as the caster brake is stepped on using the pedals or the casters are pressed, the revolutions of the casters are reduced.

In addition, there is further provided a sensing position collection part which is employed to sense and transmit the rotation angles of the foot board 3 and the base plate 12 and the upper and lower positions of the handle 17.

### ADVANTAGEOUS EFFECTS

Through the aforementioned configurations, the present invention is able to provide a virtual reality experience which is very close to an actual situation in such a way to provide a feeling, for example, a sliding, which was not implemented by a conventional device.

### BRIEF DESCRIPTION OF THE DRAWINGS

The present invention will become better understood with reference to the accompanying drawings which are given only by way of illustration and thus are not limitative of the present invention, wherein;

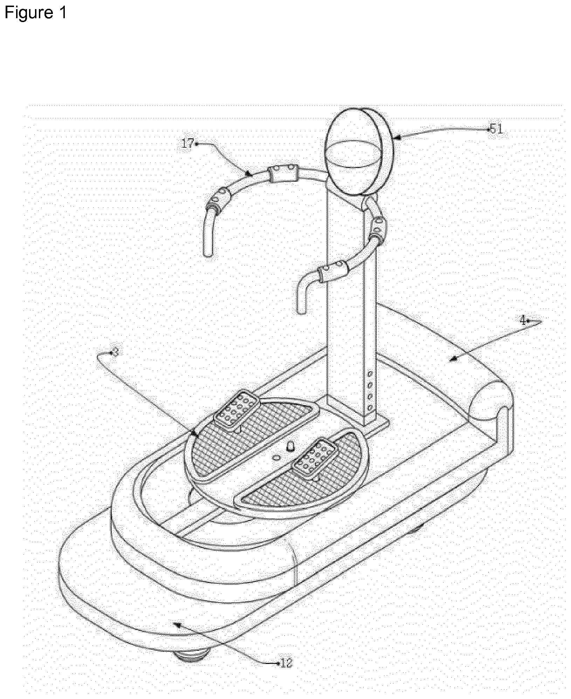

Figures 1 to 7 are views illustrating a curve cornering simulation device for providing the feeling of sliding when turning according to an embodiment of the present invention.

### MODES FOR CARRYING OUT THE INVENTION

The present invention will be described in detail with reference to the accompanying drawings. It is noted that the same components or parts in the drawings are given the same reference numbers. Throughout the descriptions, the detailed descriptions on the known functions or components related with the descriptions of the present invention will be omitted to avoid making ambiguous the subject matters of the present invention.

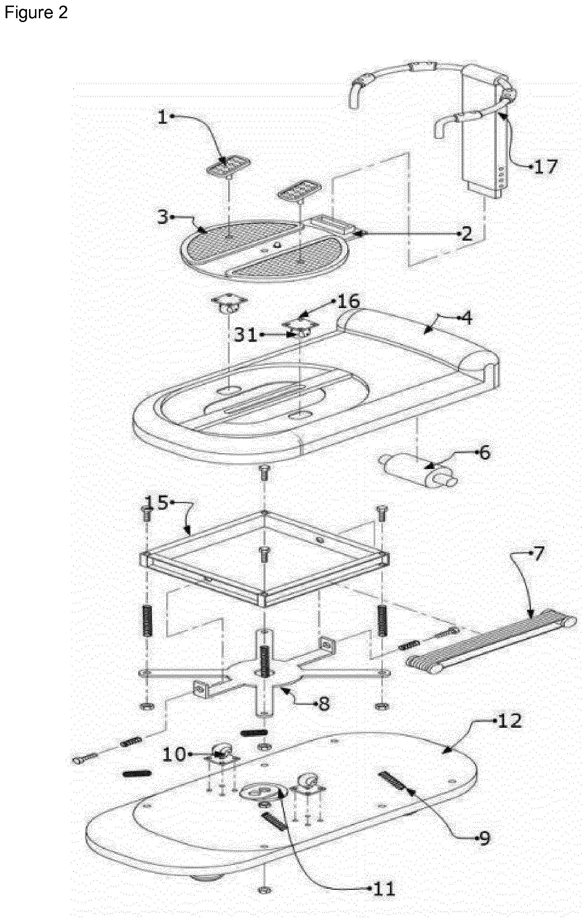

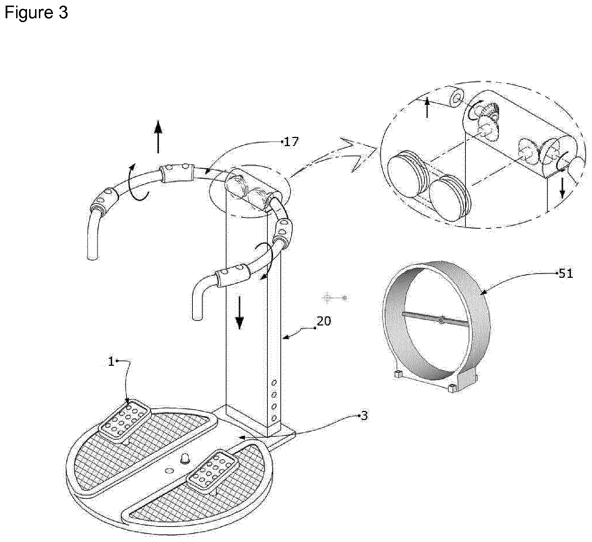

Figure 1 is a perspective view illustrating the exterior of a simulation device according to an embodiment of the present invention, and Figure 2 is a disassembled perspective view for describing an assembling correlation, and Figure 3 is a view for describing the operation of a handle. Referring to Figure 1, a foot board 3 is provided at an upper portion of a case 4, and foot pedals 1 are provided opposing to each other at both sides of the foot board 3, and a handle shaft 20 is provided at an end portion of the foot board 3, and a handle 17 is attached thereto. A user operates the device while rotating the handle 17 in a state where the user steps on the foot pedals on the foot board 3.

Figure 2 is a view illustrating the assembled state of the present invention. Describing with reference to the lower portions, a lower rotary shaft 11 is installed at a base plate 12, and an intermediate frame 8 having a plurality of arms is engaged to an upper portion of the lower rotary shaft. The intermediate frame 8 is supported by a spring 9 one end of which is engaged to the base plate, and a quadrangular frame 15 is engaged to an upper portion of the intermediate frame 8. In case of the front and rear surfaces of the quadrangular frame 15, the intermediate frame 8 is thread-engaged to the arm which faces the front and rear surfaces among the arms protruding in the radial direction from the circular center, and a support plate is engaged to a corner portion of the quadrangular frame 15. Moreover, in a base plate 12, a lower caster 10 is engaged to both sides of the lower rotary shaft 11. When engaged, it may protrude through the arms of the intermediate frame 8. The intermediate frame 8 may be configured to rotate along the lower rotary shaft or to be slanted downward or upward.

Since it is engaged to the intermediate frame 8 at the front and rear surfaces of the quadrangular frame, the quadrangular frame 15 can rotate in the axial direction or can move in the upward and downward directions about the axis at the side surface.

A track belt 7 is engaged to the quadrangular frame 15. The belt of the track belt 7 rotates by the torque transferred from an electric motor 6, and the electric motor 6 is engaged to the inner side of the case.

Moreover, the lower caster 10 may contact with the lower surface of the track belt 7, and an upper caster may contact with the upper surface of the track belt 7. The case 4 may cover the tops of the quadrangular frame 15 and the electric motor 6, and the foot board 3 may be engaged to the upper surface of the case 4, and a pair of the foot pedals 1 are disposed at the foot board 3. In this configuration, the foot pedals 1 are engaged to the pedal engaging part 16 provided at the upper surface of the case 4, and the pedal engaging part 16 may pass through the case 4, and the upper caster 31 may be attached to a lower end portion of the pedal engaging part 16, and the upper caster may contact with the track belt 7. In a state where the user stands on the foot board 3 and is holding the handle 17, when the user steps on the foot pedals 1, the rotation of the foot pedal 1 is transferred to the upper caster. The handle 17 is attached to the handle shaft 20, and the handle shaft 20 is engaged inserted in the handle engaging part 2 of the front side of the foot board 3. The track belt may be formed in a single structure or may be divided into multiple parts. It can be provided as many as the number of pedals. They are all configured to rotate with the same revolutions.

Referring to Figure 3, as for the configuration of the handle, the handle is disposed to be movable upward and downward. The motion of the handle of one side may be transferred to the handle of the other side via the gears and the belt, so the handle can move in cooperation with each other in the other direction.

The upper caster 31 is configured to rotate, and the rotational motion can be limited by a caster brake connected to the foot pedals when the user steps on the foot pedals. If the user removes his foot from the foot pedals, a recovery force may be formed by the spring provided at the caster brake.

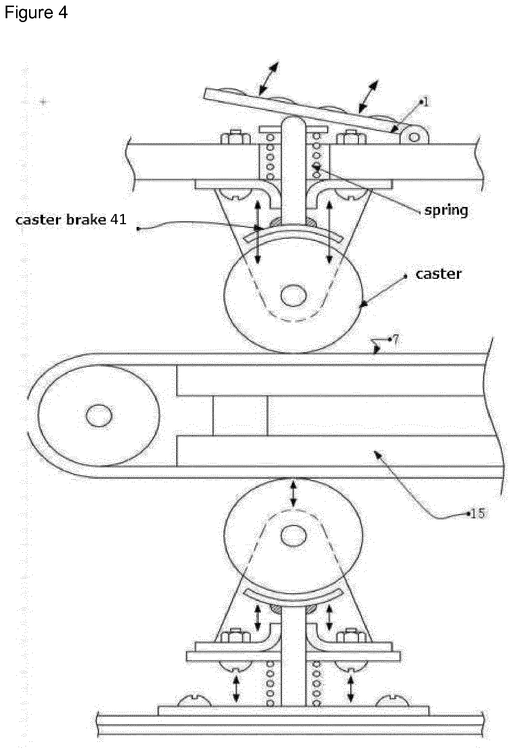

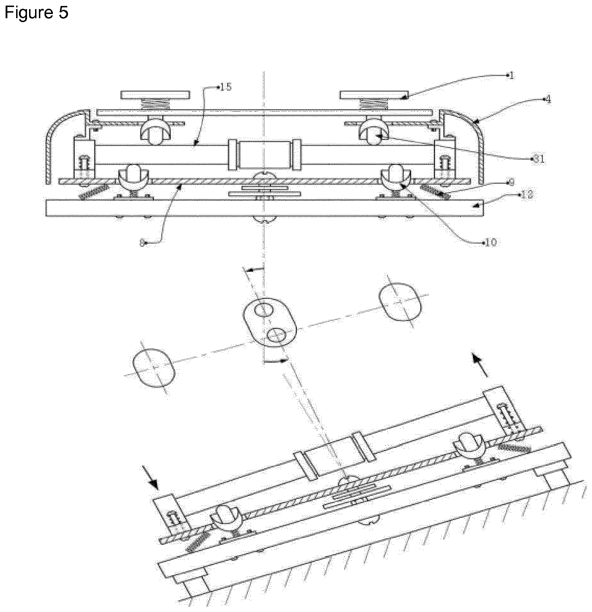

Figures 4 and 5 shows the configuration of the upper caster, the lower caster and the track belt. As illustrated therein, the upper caster 31 may face the lower caster 10 with the track belt 7 being disposed between them. The intermediate frame 8 is engaged to the upper surface of the base plate 12 by means of the lower rotary shaft 11, and the quadrangular frame 15 may be engaged to the intermediate frame 8. The intermediate frame 8 may be fixed at the lower rotary shaft 11 and may be supported by the spring 9 and near the lower rotary shaft 11. If the intermediate frame 8 is pressed downward, it can have a recovery force, and a support plate 32 may be engaged to an inner portion of the corner of the quadrangular frame 15 and can be pressed inwardly by the screw and the spring which are engaged to the inner corner portion. If the support plate 32 is pressed, it can be pushed inwardly by the spring, but may return to its original position. The support plate 32 is inserted in a hole formed at a portion of the arm of the intermediate frame 8. If a force is supplied to the intermediate frame 8 or the quadrangular frame 15, the support frame 32 can also be pressed. If the pressing force is large, the support plate may come down below the hole of the arm, and it may be gradually pressed step by step by elasticity.

Moreover, the track belt 7 is engaged to the quadrangular frame 15, and the lower surface of the track belt 7 may contact with the lower caster 10 attached to the base plate 12. Since the intermediate frame 8 is formed in such a way that the arm is extending from its center, the lower caster 10 can contact with the lower surface of the track belt 7 via the empty space where the arm is not present.

Figures 4 and 5 shows the aforementioned configuration, of which Figure 4 shows a configuration when viewing sideways, and Figure 5 shows a configuration when viewing from the front and rear surfaces.

In this configuration, if the user steps on the foot pedal 1 and rotate the handle, as illustrated in Figures 4 and 5, the upper caster 31 below the foot pedal 1 may be pressed, and if the caster brake 41 is pressed downward and interrupts the rotation of the caster, the caster may slide over the rotating track belt, so the foot board 3 can be slanted a little in the direction where the foot pedal has been stepped on. If the foot pedal is not stepped on, the caster below the foot pedal may rotate along the track belt, but the rotational motion of the caster may be limited, and if any sliding occurs, a friction noise may occur. The revolutions of both the caster may differ, and the foot pedal which has not been stepped on, may keep rotating, by which the foot board can rotate in the direction of the foot pedal which has been stepped on.

In the conventional technology, one caster is used instead of using two casters so as to change the direction of the caster, thus providing the aforementioned rotational motion. In the present invention, the foot board 3 may rotate in such a way to use two casters even though the caster does not change its direction.

In case of the forward or backward movements, the foot pedals can be pressed without rotating the handle. The forward and backward movements can be determined based on the rotating direction of the track belt 7. The rotating direction of the track belt 7 can be manipulated by the handle.

If the driver bends his upper body to the maximum in one direction or causes an deviated load through a predetermined manner, the quadrangular frame may be pressed, and the lower caster 10 contacting under the quadrangular frame may be also pressed, and consequently, the quadrangular frame may be pressed in the direction of the top of the arm of the intermediate frame 8 to which the quadrangular frame 15 has been engaged. Since the lower caster 10 is being supported by the spring, and the intermediate frame 8 is being supported by the spring 9, the aforementioned pressing force can be offset.

If the lower caster is pressed as the track belt 7 is pressed, the caster of the lower caster 10 may contact with the caster brake, and the revolution of the caster is reduced or the caster may stop, for which the caster may slide over the belt. If one caster among the casters at both sides keeps rotating, and the other caster slides or the revolution thereof is reduced, the quadrangular frame 15 may move in the direction of the caster about the base plate which is being pressed about the axis. More specifically, it will rotate in the direction where the weight has been applied, irrespective of what the foot board 3 has moved when stepping on the pedal. Through the aforementioned operation, the quadrangular frame and the intermediate frame may be deviated sideways when viewing with reference to the base plate 12 in a state where they don't touch the floor since they are being supported by the spring 9 below the same or the lower caster 10.

Even in a state where the user keeps stepping on the pedals with only his foot, it seems that the user is seeing the foot board 3, the case 4 and the base plate 12. If the user steps on the pedal, the direction of the foot board 3 may be deviated, and if the user steps on with his weight in addition to the aforementioned stepping, the direction of the case 4 can be deviated with respect to the base plate. When only the foot board 3 is rotated, it may be called a first rotation, and when the case 4 is rotated in addition to the aforementioned rotation, it may be called a second rotation.

In the conventional virtual reality experiencing device, there is an occasion wherein the foot rest corresponding to the foot board 3 might rotate. In this case, it is impossible to implement with only one foot rest the riding actions which are carried out by various driving device and brakes. As compared thereto, the present invention is able to implement the aforementioned motions in very precise ways, in particular, the motions during the rotation.

Since the conventional devices cannot implement a sliding action, the direction is changed with a discontinuous motion; however the present invention is able to implement a left and right manipulation and an action based on the weight, for which the present invention is able to provide a reality-like motion. For this, the rotation angles of the foot board 3 and the track cover 4, the upward and downward position displacements of the handle and the left and right position displacement can be sensed and operated in cooperation with the player of a game or a content, and a 6-axis gyroscope sensor can be used.

The system according to the present invention operates at 100 volts to 240 volts and is able to adjust the speed of the track belt 7. If a start button is pressed in the stopped state, the system rotates at the minimum speed, and the speed can be increased or decreased. This device is installed at the handle. A sensor is installed at the roller of the belt so as to sense the rotation speed of the track belt, by which it is possible to control that the rotation speed of the track belt does not exceed a predetermined speed.

Moreover, wind can be blow toward the user in accordance with a control of a separate air blowing controller. The air blowing intensity can be controlled in cooperation with the rotation speed of the track belt. As the rotation speed increases, the air blowing intensity can be increased, through which the user can feel more reality. The electric power may be interrupted if the stop button is activated or it deviates from the stopper.

Figures 4 and 5 show an engaged state of the upper caster, the lower caster and the track belt. If the user steps on the foot pedal 1, the caster brake 41 may come down and interrupt the caster from rotating, and if the user removes his foot from the foot pedal, the caster brake may rise by the action of the spring, so the caster returns. If the caster brake 41 is operating, the caster will slide over the track belt 7 and may start moving. In this case, the revolutions of the wheels (casters) at both sides may differ from each other, so the brake may operate inclined in one direction.

The lower caster is contacting with the lower surface of the track belt 7. If it is pressed downward, and the caster is not rotated by the operation of the caster brake 41, the caster of one side is pushed and inclined toward the pushed side.

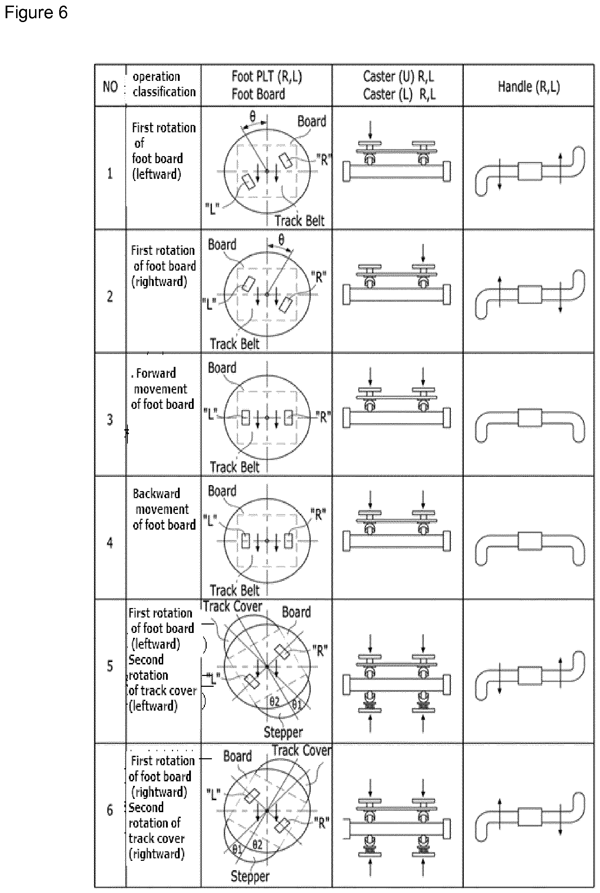

Figure 6 shows the motion of the foot board during the operations of the handle and the foot pedal and when the deviated weight is applied.

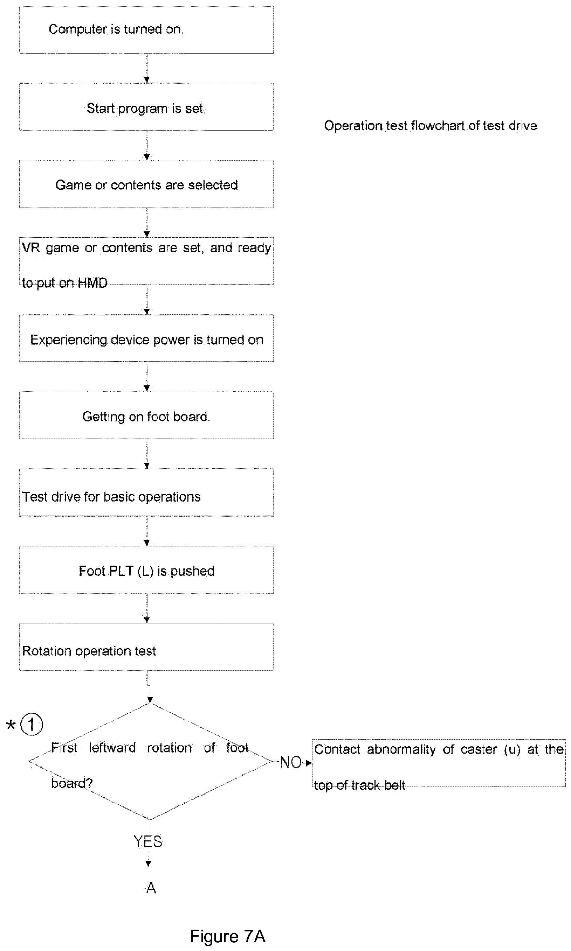

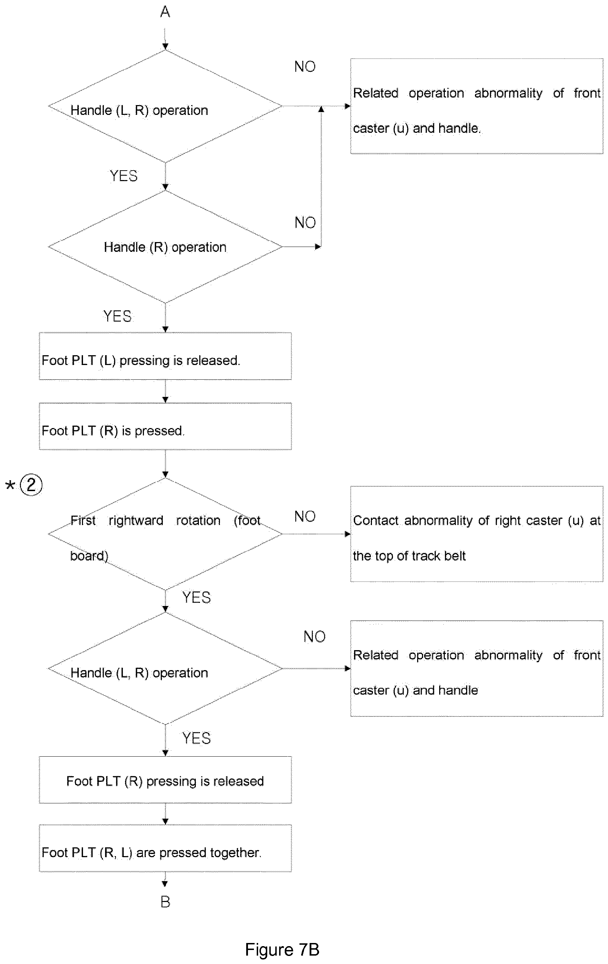

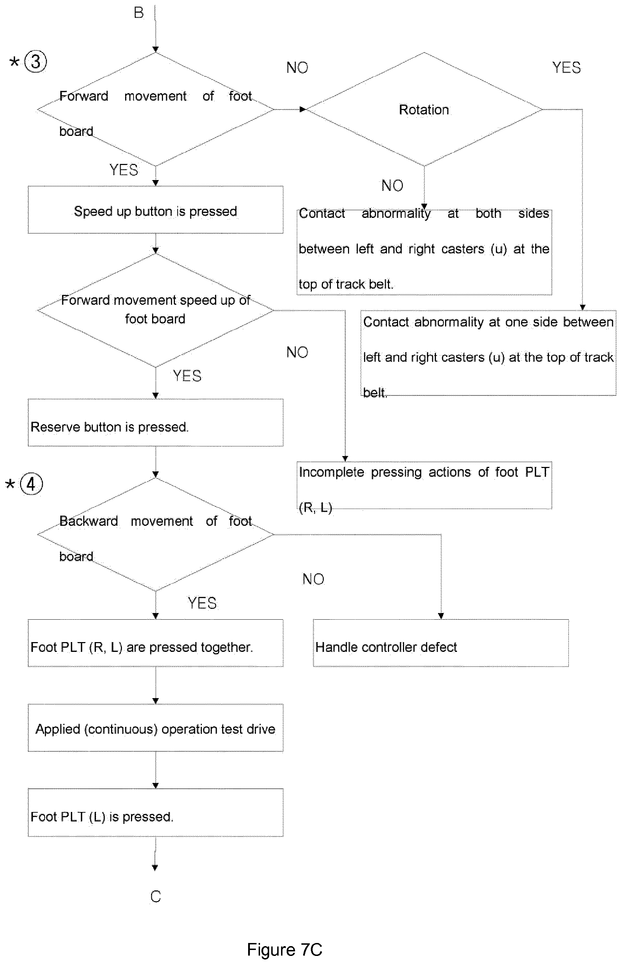

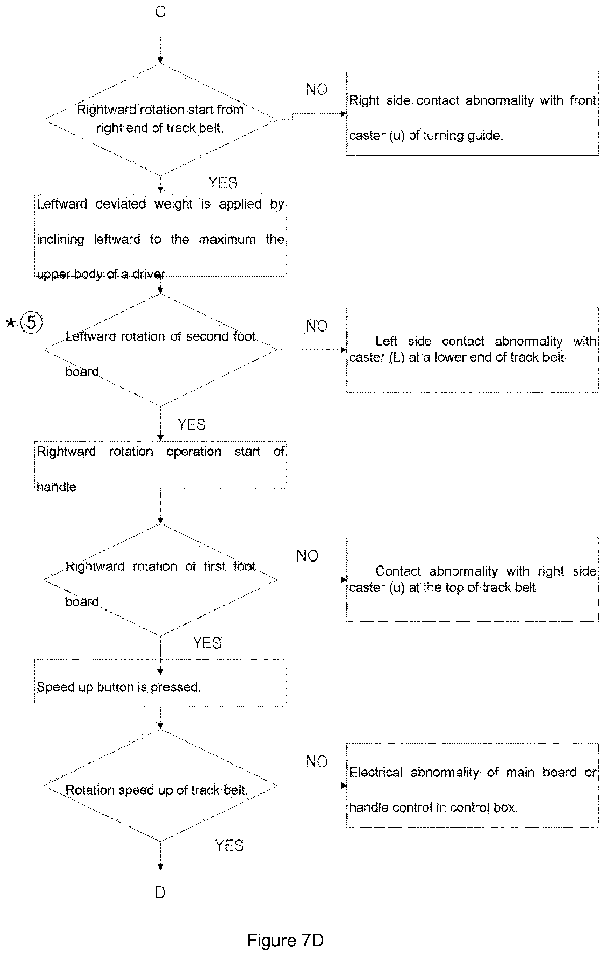

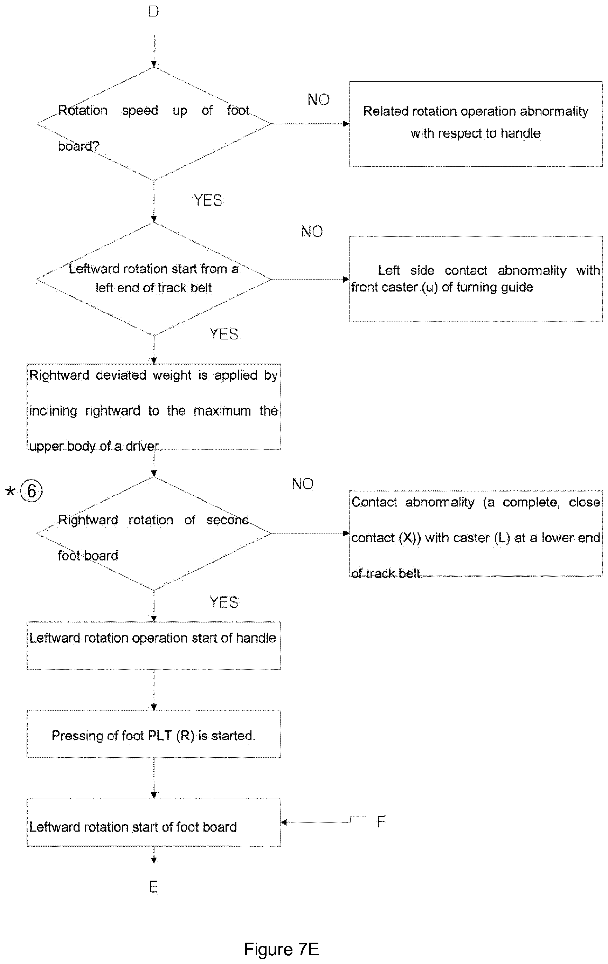

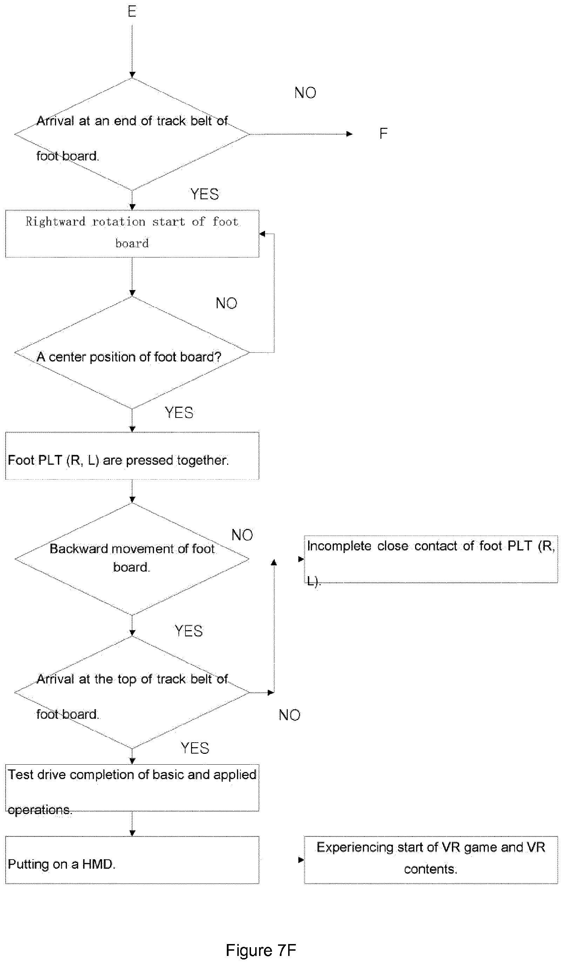

Figure 7 is a flow chart for describing the operation of the device according to the present invention. Figure 7 shows an operation test before the test drive. The electric power is turned on by selecting the device according to the present invention and the contents which use the device. The user can get on the foot board after he has putted on the HMD (Head Mounted Device) which supports a virtual reality.

When the user steps on the left pedal, if the foot board 3 rotates leftward, it means that the foot board 3 operates normally. Otherwise, it needs to check out if there is a contact abnormality at the caster which contacts with the top of the track belt. If it operates normally, it needs to check out if the handle can rotate leftward and rightward, and otherwise, it is checked that there is an abnormality in a related operation between the upper caster and the handle. If it is normal, the pressing of the left foot pedal is released, and the same checking will be carried out with respect to the right foot pedal. If the check on the abnormality with respect to the foot pedals is completed, the forward and backward movements of the foot board are checked out. If the forward movement does not work in accordance with the forward movement setting, and the foot board rotates, it is checked if there is a contact abnormality at the caster of one side among the left and right casters at the top of the track belt, and otherwise, it is checked if there is a contact abnormality at both sides of the left and right sides at the top of the track belt.

In case of the backward movement, it is checked out in the same way as the forward movement. The upper body of the driver is bent to the maximum in the leftward direction so as to provide the leftward deviated weight, and then the second left rotation of the foot board is checked. If the second left rotation does not occur, it is checked if there is a left side contact abnormality at the lower caster, and it needs to check out if the right rotation of the first foot board occurs while rotating the handle in the rightward direction, and the related operations with respect to the handle are checked out while increasing the speed of the track belt.

Thereafter, it is checked out if the second right rotation of the foot board occurs in a state where the rightward deviated weight has been applied by inclining to the maximum the upper body of the driver, and it is checked out if the normal operation occurs in response to the leftward rotation of the handle and the pressing of the foot pedal. If all the checks are completed, the user puts on the HMD (Head Mounted Display) and starts experiencing a virtual reality game or virtual reality contents.

As the present invention may be embodied in several forms without departing from the spirit or essential characteristics thereof, it should also be understood that the above-described examples are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

**Legends of reference number**

| | |
|---|---|
| 1: Foot pedal | 3: Foot board |
| 4: Case | 6: Electric motor |
| 7: Track belt | 8: Intermediate frame |
| 9: Spring | 10: Lower caster |
| 11: Lower rotary shaft | 12: Base plate |
| 15: Quadrangular frame | 16: Pedal engaging part |
| 17: Handle | |
| 31: Upper caster | |
| 51: Air blowing unit | |

## Claims

1. A curve cornering simulation device for providing the feelings of sliding and a friction when turning, comprising:
a foot board (3) rotatable with respect to a rotary shaft;
a driven track belt (7) configured to move in use at the outer sides of a pair of rollers a pair of rollers; **characterized by** the curve cornering simulation device further comprising: a pair of foot pedals (1) installed on the foot board (3) with the rotary shaft being disposed between the foot pedals (1); and
a caster (31) with a caster brake (41) provided above the caster (31) and disposed under the foot pedals (1), the caster (31) is configured for rotation on the top of the moving track belt (7), such that when, in use, a user steps on the foot pedal (1), the caster brake (41) comes down and interrupts the rotation of the caster (31) on the top of the track belt (7), such that the caster (31) and the foot board (3) rotate with respect to the rotary shaft in the direction of the foot pedal (1) being stepped on.

2. The device of claim 1, wherein the track belt (7) is on the same straight line as the rotary shaft of the foot board (3) and is placed rotatable on a lower rotary shaft (11) which is not engaged, and a pair of lower casters (10) is installed at a lower end portion of the track belt (7) with the lower rotary (11) shaft being disposed between the lower casters (10), by which it is pressed in the direction where a deviated weight occurs, thus being rotated.

3. The device of claim 1, wherein the track belt (7) contacts an end portion of each of a pair of lower casters (10) installed at the top of a base plate (12), and a pair of lower casters (10) is installed at a portion which is symmetrical about a lower rotary shaft (11) and is pressed in the direction where a deviated weight occurs, thus being rotated.

4. The device of claim 3, wherein the rotary shaft is on the same axis as the lower rotary shaft (11).

5. The device of either claim 2 or claim 4, wherein an intermediate frame (8) with a plurality of arms is placed on the lower rotary shaft (11), and a quadrangular frame (15) is engaged to the intermediate frame (8), and the track belt (7) is engaged to the quadrangular frame (15), and the lower casters (10) and a spring (9) support under the intermediate frame (8), and if a deviated weight is applied, the lower casters (10) are pressed by the deviated weight.

6. The device of claim 5, wherein a handle engaging part (2) is provided at an end portion of the foot board (3), and a handle shaft (20) stands at the handle engaging part (2), and a handle (17) is provided at an end of the handle shaft (20), and the handle (17) is configured to move upward and downward, and both ends of the handle (17) are configured to move in different directions in cooperation with each other.

7. The device of claim 6, wherein the lower casters (10) and the upper casters (31) are configured to rotate in such a way that the casters (10, 31) are engaged to the inner sides of two support shafts, and the caster brake (41) to which a spring is attached in the direction where the support shaft is engaged, is configured to move in the direction of the casters, and if the caster brake (41) contacts the casters as the caster brake (41) is stepped on using the foot pedals (1) or the casters are pressed, the revolutions of the casters are reduced.

8. The device of claim 7, further comprising a sensing position collection part which is employed to sense and transmit the rotation angles of the foot board (3) and the base plate (12) and the upper and lower positions of the handle (17).

9. The device of claim 8, further comprising an air blowing unit (51) which is employed to blow wind toward the user based on the speed of the track belt (7).

## Patentansprüche

1. Kurvenfahrtsimulationsvorrichtung zur Bereitstellung des Gefühls von Gleiten und einer Haftung in Kurven, umfassend:
eine Fußplatte (3), die in Bezug auf eine Drehwelle drehbar ist;
ein angetriebenes Laufband (7), das konfiguriert ist, sich in Verwendung an den Außenseiten eines Paares von Walzen zu bewegen; **gekennzeichnet durch** die Kurvenfahrtsimulationsvorrichtung, die weiter umfasst: ein Paar von Fußpedalen (1), das an der Fußplatte (3) installiert ist, wobei die Drehwelle zwischen den Fußpedalen (1) angeordnet ist; und
eine Rolle (31) mit einer Rollenbremse (41), die über der Rolle (31) bereitgestellt und unter den Fußpedalen (1) angeordnet ist, die Rolle (31) zur Drehung an der Oberseite des sich bewegenden Laufbands (7) konfiguriert ist, sodass, wenn in Verwendung ein Anwender auf das Fußpedal (1) tritt, die Rollenbremse (41) gesenkt wird und die Drehung der Rolle (31) auf der Oberseite des Laufbands (7) unterbricht, sodass die Rolle (31) und die Fußplatte (3) in Bezug auf die Drehwelle in der Richtung des Fußpedals (1) drehen, auf das es getreten wird.

2. Vorrichtung nach Anspruch 1, wobei das Laufband (7) auf derselben geraden Linie wie die Drehwelle der Fußplatte (3) liegt und drehbar auf einer unteren Drehwelle (11) platziert ist, mit de r nicht eingegriffen wird, und ein Paar unterer Rollen (10) bei einem unteren Endabschnitt des Laufbands (7) installiert ist, wobei die untere Drehwelle (11) zwischen den unteren Rollen (10) angeordnet ist, durch welche sie in die Richtung gepresst wird, wo ein abweichendes Gewicht auftritt, wodurch sie gedreht wird.

3. Vorrichtung nach Anspruch 1,
wobei das Laufband (7) mit einem Endabschnitt jeder eines Paares von unteren Rollen (10) in Kontakt ist, das auf einer Basisplatte (12) installiert ist, und ein Paar von unteren Rollen (10) bei einem Abschnitt installiert ist, der um eine untere Drehwelle (11) symmetrisch ist und in die Richtung gepresst wird, wo ein abweichendes Gewicht auftritt, wodurch es gedreht wird.

4. Vorrichtung nach Anspruch 3, wobei die Drehwelle auf derselben Achse wie die untere Drehwelle (11) liegt.

5. Vorrichtung nach Anspruch 2 oder Anspruch 4, wobei ein Zwischenrahmen (8) mit einer Vielzahl von Armen auf der unteren Drehwelle (11) platziert ist und ein rechteckiger Rahmen (15) mit dem Zwischenrahmen (8) eingreift und das Laufband (7) mit dem rechteckigen Rahmen (15) eingreift und die unteren Rollen (10) und eine Feder (9) unter dem Zwischenrahmen (8) stützen und falls ein abweichendes Gewicht angelegt wird, die unteren Rollen (10) durch das abweichende Gewicht gepresst werden.

6. Vorrichtung nach Anspruch 5, wobei ein Griffeingriffsteil (2) an einem Endabschnitt der Fußplatte (3) bereitgestellt ist und eine Griffwelle (20) an einem Griffeingriffsteil (2) steht und ein Griff (17) an einem Ende der Griffwelle (20) bereitgestellt ist und der Griff (17) konfiguriert ist, sich nach oben und unten zu bewegen, und beide Enden des Griffs (17) konfiguriert sind, sich in Zusammenarbeit miteinander in unterschiedlichen Richtungen zu bewegen.

7. Vorrichtung nach Anspruch 6, wobei die unteren Rollen (10) und die oberen Rollen (31) konfiguriert sind, auf solch eine Weise zu drehen, dass die Rollen (10, 31) mit den Innenseiten von zwei Stützwellen eingreifen, und die Rollenbremse (41), an der eine Feder in der Richtung befestigt ist, wo mit der Stützwelle eingegriffen wird, konfiguriert ist, sich in der Richtung der Rollen zu bewegen, und falls die Rollenbremse (41) mit den Rollen in Kontakt gelangt, wenn unter Verwendung der Fußpedale (1) auf die Rollenbremse (41) getreten wird oder die Rollen gepresst werden, die Umdrehungen der Rollen verringert werden.

8. Vorrichtung nach Anspruch 7, weiter umfassend ein Erfassungspositionssammelteil, das eingesetzt wird, um die Drehwinkel der Fußplatte (3) und der Basisplatte (12) und die oberen und unteren Positionen des Griffs (17) zu erfassen und zu übertragen.

9. Vorrichtung nach Anspruch 8, weiter umfassend eine Gebläseeinheit (51), die eingesetzt wird, um Wind basierend auf der Geschwindigkeit des Laufbands (7) hin zum Anwender zu blasen.

## Revendications

1. Dispositif de simulation de virage en courbe pour fournir les sensations de glissement et d'une friction lors du virage, comprenant :
un plancher (3) rotatif par rapport à un arbre de rotation ;
une courroie formant piste entraînée (7) configurée pour se déplacer durant l'utilisation au niveau des côtés extérieurs d'une paire de rouleaux; **caractérisé en ce que** le dispositif de simulation de virage en courbe comprend en outre : une paire de pédales (1) installées sur le plancher (3) avec l'arbre de rotation qui est disposé entre les pédales (1) ; et
une roulette (31) avec un frein de roulette (41) disposé au-dessus de la roulette (31) et disposé sous les pédales (1), la roulette (31) est configurée en rotation sur le dessus de la courroie formant piste mobile (7), de manière que, durant l'utilisation, un utilisateur écrase la pédale (1), le frein de roulette (41) descende et interrompe la rotation de la roulette (31) sur le dessus de la courroie formant piste (7), de manière que la roulette (31) et le plancher (3) tournent par rapport à l'arbre de rotation dans la direction de la pédale (1) qui est écrasée.

2. Dispositif selon la revendication 1, dans lequel la courroie formant piste (7) est sur la même ligne droite que l'arbre de rotation du plancher (3) et est placée de manière rotative sur un arbre de rotation inférieur (11) qui n'est pas engagé, et une paire de roulettes inférieures (10) est installée au niveau d'une portion d'extrémité inférieure de la courroie formant piste (7) avec l'arbre de rotation inférieur (11) qui est disposé entre les roulettes inférieures (10), par lesquelles il est pressé dans la direction où un poids dévié se produit, en étant ainsi pivoté.

3. Dispositif selon la revendication 1,
dans lequel la courroie formant piste (7) est en contact avec une portion d'extrémité de chacune d'une paire de roulettes inférieures (10) installées sur le dessus d'une plaque de base (12), et une paire de roulettes inférieures (10) est installée au niveau d'une portion qui est symétrique par rapport à un arbre de rotation inférieur (11) et est pressée dans la direction où un poids dévié se produit, en étant ainsi pivotée.

4. Dispositif selon la revendication 3, dans lequel l'arbre de rotation est sur le même axe que l'arbre de rotation inférieur (11).

5. Dispositif selon la revendication 2 ou la revendication 4, dans lequel un châssis intermédiaire (8) avec une pluralité de bras est placé au niveau de l'arbre de rotation inférieur (11), et un châssis quadrangulaire (15) est engagé avec le châssis intermédiaire (8), et la courroie formant piste (7) est engagée avec le châssis quadrangulaire (15), et les roulettes inférieures (10) et un support à ressort (9) sous le châssis intermédiaire (8) et, si un poids dévié est appliqué, les roulettes inférieures (10) sont pressées par le poids dévié.

6. Dispositif selon la revendication 5, dans lequel une partie d'engagement de guidon (2) est disposée au niveau d'une portion d'extrémité du plancher (3), et un axe de guidon (20) se dresse au niveau de la partie d'engagement de guidon (2), et un guidon (17) est disposée à une extrémité de l'axe de guidon (20), et le guidon (17) est configuré pour se déplacer vers le haut et vers le bas, et les deux extrémités du guidon (17) sont configurées pour se déplacer dans des directions différentes en coopération l'une avec l'autre.

7. Dispositif selon la revendication 6, dans lequel les roulettes inférieures (10) et les roulettes supérieures (31) sont configurées pour tourner de telle manière que les roulettes (10, 31) soient engagées avec les côtés intérieurs de deux arbres de support, et le frein de roulette (41), auquel un ressort est fixé dans la direction dans laquelle l'arbre de support est engagé, est configurée pour se déplacer dans la direction des roulettes et, si le frein de roulette (41) entre en contact avec les roulettes alors que le frein de roulette (41) est écrasé en utilisant les pédales (1) ou les roulettes sont pressées, les révolutions des roulettes sont réduites.

8. Dispositif selon la revendication 7, comprenant en outre une partie de collecte de position de détection qui est utilisée pour détecter et transmettre les angles de rotation du plancher (3) et de la plaque de base (12) et les positions supérieure et inférieure du guidon (17).

9. Dispositif selon la revendication 8, comprenant en outre une unité de soufflage d'air (51) qui est utilisée pour souffler du vent vers l'utilisateur sur la base de la vitesse de la courroie formant piste (7).
